(19) **Europäisches Patentamt / European Patent Office / Office européen des brevets**

(11) **EP 4 036 929 A1**

(12) # EUROPEAN PATENT APPLICATION

(43) Date of publication:
**03.08.2022 Bulletin 2022/31**

(21) Application number: **22154070.1**

(22) Date of filing: **28.01.2022**

(51) International Patent Classification (IPC):
**G16H 40/20** (2018.01)     **G16H 50/30** (2018.01)
**G16H 50/80** (2018.01)

(52) Cooperative Patent Classification (CPC):
**G16H 50/30; G16H 40/20; G16H 50/80**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.02.2021 US 202117164242**

(71) Applicant: **Eaton Intelligent Power Limited
Dublin 4 (IE)**

(72) Inventors:
• **SHYU, Brian**
  **McKees Rock, 15136 (US)**
• **BROCKETT, Neil**
  **Kerry, V31Y102 (IE)**
• **RYAN, Padhraig**
  **Wicklow (IE)**
• **ZUCCHETTO, Daniel**
  **Dublin, D04CH66 (IE)**

(74) Representative: **Wagner & Geyer
Partnerschaft mbB
Patent- und Rechtsanwälte
Gewürzmühlstrasse 5
80538 München (DE)**

(54) **WEARABLE PERSONAL PROTECTION SYSTEMS AND METHODS OF ASSESSING PERSONAL HEALTH RISK IN AN ENVIRONMENT**

(57) A method of assessing health risk in an environment is provided. The method is implemented with at least one health risk assessment computing device in communication with at least one environmental sensor and at least one proximity monitoring assembly each including a proximity sensor. The method includes computing an environmental risk score based on environmental data received from the at least one environmental sensor, and estimating a distance between the at least one proximity monitoring assembly and another proximity monitoring assembly based on proximity data received from the at least one proximity monitoring assembly. The method also includes comparing the estimated distance with a proximity threshold corresponding to the environmental risk score, and recommending personal protection measures based on the comparison and the environmental risk score.

FIG. 1A

EP 4 036 929 A1

**Description**

BACKGROUND

The field of the disclosure relates generally to computer-implemented personal protective equipment systems, and more particularly to computer-implemented wearable personal protection systems and methods of proximity-based and environmentally-based health risk assessment in an environment.

A variety of different types of personal protective equipment (PPE) exist that is required by a host of healthcare, industrial, utility, and trade workers to provide a degree of protection from known risks in the hazardous environments where they work. Conventionally, such PPE is not designed to protect workers against risks of airborne disease that are now of much concern in the workplace. Risk of transmission of airborne diseases can be affected positively or negatively by environmental factors such as temperature, humidity, and ventilation that are not easily assessed by human persons or accounted for in existing PPE protocols.

Computer-implemented monitoring systems exist that intelligently incorporate sensors to create a degree of situational awareness of risks posed to workers, but known systems of this type generally lack capability to detect specific health risk and PPE issues associated with particular individuals in a particular environment, and specifically lack capability to intelligently assess risks of airborne disease transmission. Improvements are therefore desired.

BRIEF DESCRIPTION OF THE DRAWINGS

Non-limiting and non-exhaustive embodiments are described with reference to the following Figures, wherein like reference numerals refer to like parts throughout the various drawings unless otherwise specified.

FIG. 1A is a schematic diagram of an exemplary health risk assessment system.

FIG. 1B is an exemplary embodiment of the health risk assessment system shown in FIG. 1A.

FIG. 1C is a schematic diagram of a proximity monitoring assembly in the health risk assessment system shown in FIG. 1A.

FIG. 2A is a flow chart of an exemplary method of assessing health risk in an environment.

FIG. 2B is a flow chart of an exemplary embodiment of the method shown in FIG. 2A.

FIG. 3 is a schematic diagram of a user computing device.

FIG. 4 is a schematic diagram of a server computing device.

DETAILED DESCRIPTION

In order to understand the inventive concepts described below to their fullest extent, set forth below is a discussion of the state of the art and certain longstanding problems pertaining to personal wellness and personal protection equipment (PPE), followed by systems, assemblies, and methods addressing longstanding problems in the art.

It is a practical reality, in certain industries, that exposure of at least some workers to hazardous, or potentially hazardous, working conditions cannot be avoided. As one example, workers in the electrical industry, and more specifically those working in and around electrical power systems, must be trained in the appropriate use of PPE to mitigate possible electrical hazards with which they may face.

Aside from hazards associated with electrical shock and electrocution, electrical arc flash incidents are of particular concern. Electrical arcing, or current flow between two or more separated energized conductors, may be experienced when installing, servicing, and maintaining electrical systems. Arcing may occur from electrical fault conditions and can release significant amounts of concentrated radiant energy at the point of arcing in a fraction of a second, resulting in high temperatures that may burn persons exposed to them. Additionally, arcing conditions may produce pressure blasts that are more than sufficient to knock nearby workers off their feet, and shrapnel may be generated by the blast.

Arcing in an electrical power system may suddenly arise in various scenarios that cannot be reliably predicted. For example, insulation failure of components used in electrical systems, including but not limited to cables that interconnect electrical components and equipment may precipitate arcing, as well as a build-up of dust, impurities and corrosion on insulating surfaces. Sparks generated during operation of circuit breakers, during replacement of fuses, and closing electrical connections on faulted lines may also produce an arc. Damage to components and equipment from rodents and pest infestations may result in arcing conditions. Finally, arcing may be the result of unpredictable scenarios of human error such as dropping a tool onto energized conductors, accidental or incidental contact with energized components or equipment, and improper work procedures or mistake in following a procedure to completing a task.

Accordingly, PPE that is adequate or sufficient to provide at least a minimum level of protection to persons against potential electrical hazards has been developed for practically the entire human body, such as for example, electric shock, arc flash and arc blast. Persons wearing such personal protective equipment may be reasonably protected from incidental contact with energized conductors and potentially hazardous arc flash incidents and such PPE may avoid or reduce the likelihood of serious injury if such an arc flash incident occurs. Examples of PPE items may include a hard hat, a face shield, a flame resistant neck protector, ear protectors, a Nomex™ suit, insulated rubber gloves with leather protectors, and insulated leather footwear. Insulated tools may also be provided to complete certain tasks. Such personal protective equipment may be fabricated from various materials to provide, among other things, thermal insulation protection to prevent severe burns to human flesh during high temperature arcing conditions, and to mitigate pressure blasts and shrapnel to avoid life-threatening wounds to a worker's head and torso if arcing conditions were to occur. Different grades of PPE are available to protect against varying degree of risk presented. For example, in the case of electrical fuses that need replacement under energized circuit conditions, fuses of higher electrical ratings may pose a greater risk than fuses of lower electrical ratings, and different amounts or types of personal protective equipment may be required for replacing one fuse, for example, than for replacing another fuse.

Similar considerations exist for other types of hazardous environments rendering similar PPE items desirable for use such as, for example only, petroleum refineries, petrochemical plants, grain silos, wastewater and/or treatment facilities, or other industrial facilities in which sustained or volatile conditions in the ambient environment may be present and may present a heightened risk of fire or explosion and/or a potential exposure to caustic chemicals and substances. Various different grades of PPE are available, which may be similar to or different from the grades of PPE designed for electrical hazards, to meet different risks posed by different situations.

In addition to hazardous locations discussed above, so-called harsh locations also require specific focus in the design of PPE and risk assessment systems used therewith. Harsh locations may entail corrosive elements and the like in the atmosphere that are not necessarily explosive and/or are subject to temperature cycling, pressure cycling, shock and/or mechanical vibration forces that are typically not present in non-harsh operating environments. Of course, some locations in which PPE and risk assessment systems are desirably employed are both harsh and hazardous by nature, and are therefore PPE and risk assessment systems designed for various operating conditions that typical PPE and risk assessment systems for other uses are not suitable.

In the healthcare environment, PPE items have been used to protect doctors and nurses in the treatment of patients having conditions that present health risks to healthcare providers when performing certain procedures. Different grades of PPE are available to meet different risks posed by different healthcare procedures. Paramedics, Emergency Medical Technicians (EMTs), Law Enforcement Offices, Firefighters and other emergency responders, as well as military personnel also have PPE items and protocols for responding to certain situations.

The onset of COVID-19 has raised new concerns and demands for health risk assessments and the proper PPE in environments that, prior to COVID-19, were generally not considered "hazardous" in a manner demonstrating a need for PPE. Such environments include areas of industrial facilities that are isolated from conventionally defined hazards, healthcare facilities and areas of healthcare facilities that were not previously considered to present high risk scenarios, elementary schools, middle schools, high schools, colleges and universities, offices and businesses of all types, shops and retail establishments, dining establishments, churches, and entertainment venues. Desirable PPE items are therefore prolifically present in these environments.

In the COVID era, individual personal wellness is an important consideration to ensure that no transmission of the virus occurs to nearby persons. In general, persons having COVID symptoms are strongly advised not to interact with other persons, but in some instances a person may have symptoms without necessarily realizing it or may be contagious without onset of any symptoms.

Airborne disease can be transmitted when people are in close proximity. Social distancing and masking are another important consideration to address risks posed by other persons possibly having the COVID virus or other conditions that can be contagiously spread or communicated to others. Face shields may suffice for the mask requirement in certain environments, but may be inadequate in protecting against the virus in other environments. Proximity sensing and contact tracing technologies have emerged to monitor social distancing aspects and collect information that may be helpful to maintain an outbreak of illness, but they are disadvantaged in some aspects for certain hazardous environments. For instance, smart-phone based contact tracing apps are of no aid in environments where smart phones are prohibited. Known contact tracing apps also operate independently of PPE systems and lack capability to assess wellness in a proactive manner.

Known PPE systems do not consider the effects of environmental factors, such as temperature, humidity, and ventilation, in the health risk of airborne disease before recommending PPE and monitoring PPE compliance either. The transmission of airborne disease is also affected by environmental factors. In some environments, it is relatively safe to wear a face shield as PPE, while in some environments, wearing a face mask is required for added protection. Further, in industrial settings, such as harsh and hazardous environments, health risks posed by hazardous or potentially hazardous working conditions to the workers such as dangerous emissions and particles and potential damages to the machinery and buildings are also influenced by environmental factors. Therefore, there is a need for systems to dynamically assess health risk and guide people with appropriate PPE for various environments.

For the reasons above, effective health risk assessment systems are needed to more intelligently address health risks in an environment and provide corresponding PPE recommendations that are COVID related and non-COVID related but nonetheless implicate important wellness concerns to varying degrees.

The health risk assessment system of the disclosure may be equally applicable to any of the areas listed above, or other areas that present similar issues or concerns, which are deemed hazardous in a non-conventional way solely because of COVID issues or other pandemic or epidemic outbreaks that compel a use of PPE, and/or conventional areas deemed hazardous in a conventional way due to risks such as shock, blasts, impact, fire, explosion, chemical burns, and all sorts of undesirable exposure to potentially harmful elements.

The assemblies, systems, and methods disclosed herein include environmental sensors and proximity sensors, provide communications, alert, and/or feedback to the wearer or other individuals to enhance safety, and provide recommendations on PPE. The description of virus transmission such as transmission of COVID virus is for illustration purposes only. The systems and methods may be applied to assessing health risk in an environment of airborne diseases caused by any contagions, particles, emissions, hazardous or potentially hazardous working conditions, or a combination therein. Method aspects will be in part apparent and in part explicitly discussed in the following description

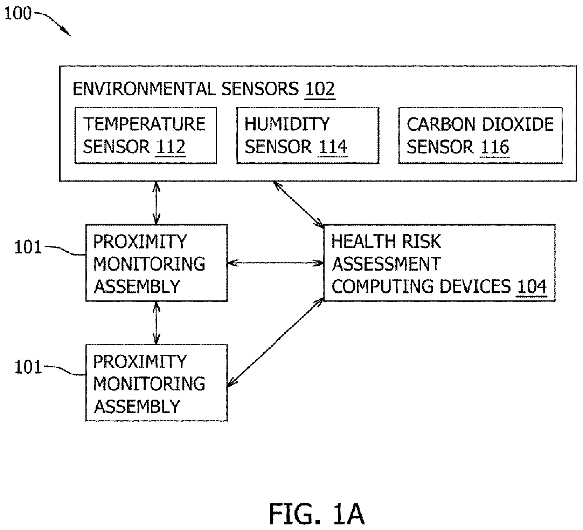

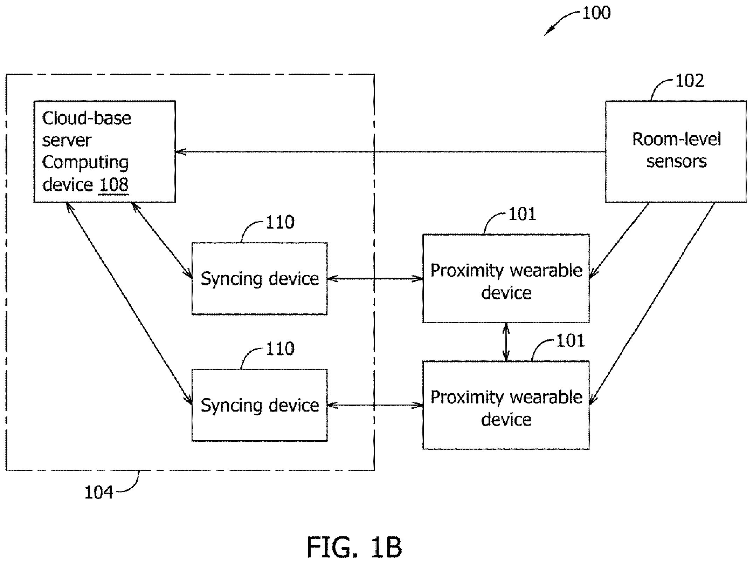

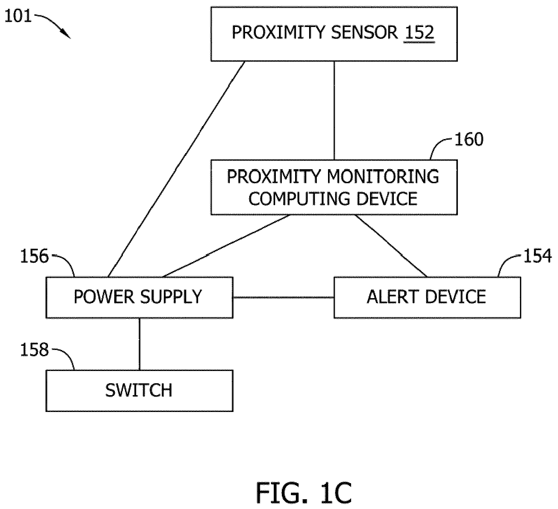

FIGs. 1A-1C show an exemplary health risk assessment system 100 (FIGs. 1A and 1B) and an exemplary proximity monitoring assembly 101 (FIG. 1C). FIG. 1A is a schematic diagram of the health risk assessment system 100. FIG. 1B is an exemplary embodiment of the health risk assessment system 100. FIG. 1C is a schematic diagram of the proximity monitoring assembly 101. The health risk assessment system 100, proximity monitoring assemblies 101, and methods described herein are used to assess health risks of external hazard, recommend PPE, and to enhance safety of the workers.

The health risk assessment system 100 includes at least one proximity monitoring assembly 101 and at least one environmental sensor 102. The environmental sensor 102 and the proximity monitoring assembly 101 may be separate devices or may be integrated into one device. The health risk assessment system 100 may further include a health risk assessment computing device 104. The health risk assessment computing device 104 may be located separately from the proximity monitoring assembly 101 and the environmental sensors 102. In some embodiments, the health risk assessment computing device 104 is a server computing device, and may be cloud-based. In other embodiments, the health risk assessment computing device 104 is a user computing device, such as a mobile device or a personal computer. In yet other embodiments, the health risk assessment computing devices 104 include a server computing device 108 and a user computing device 110 (FIG. 1B). In one example, the health risk assessment computing device 104 is integrated with one or more of the environmental sensors 102 and/or one or more of the proximity monitoring assemblies 101. The environmental sensors 102 communicate with the proximity monitoring assemblies 101, and with the health risk assessment computing device 104. The environmental sensors 102 and/or the proximity monitoring assemblies 101 may upload signals detected by them and/or data stored in them to the health risk assessment computing device 104, and/or receive commands from the health risk assessment computing device 104. The communication may be periodic or in real time.

The environmental sensors 102 may include a temperature sensor 112 that measures an ambient temperature of an environment, such as a room. The environmental sensors 102 may include a humidity sensor 114 that measure the humidity of the environment. In some embodiments, the environment sensors 102 may further include a carbon-dioxide ($CO_2$) sensor 116 that measures the level of carbon-dioxide in the environment. The environmental sensors 102 may be mounted in the environment such as on a wall of the room. In some embodiments, the environmental sensors 102 may be integrated with the proximity monitoring assembly 101 such that the environment sensors 102 measure the environment surrounding the wearer of the proximity monitoring assembly 101. Other sensors, such as environmental sensors that measure pressure and oxygen may be included in the health risk assessment system 100 to enable the health risk assessment system 100 to function as described herein.

In some embodiments, the health risk assessment system 100 also includes a location sensor (not shown). The location sensor may be integrated with one or more of the environmental sensors 102 and/or one or more of the proximity monitoring assembly 101. The location sensor may also be integrated with one or more of the health risk assessment computing devices, such as a mobile device. The location sensor may include a global position system (GPS) receiver or a wireless beacon. The location sensor determines a location of the location sensor, which is used to determine whether an individual such as a wearer of the proximity monitoring assembly 101 is indoors or outdoors. For example, the location sensor is worn by the individual and the location sensor determines whether the individual is indoors or outdoors based on the location data. In another example, the location sensor is fixed at one location, and measures the relative location or distance between the location sensor and the individual and determines whether the individual is indoors or outdoors based on the relative location or distance.

In one embodiment, one or more of the health risk assessment computing devices 104 include a processor-based microcontroller including a processor and a memory device wherein executable instructions, commands, and control algorithms, as well as other data and information needed to satisfactorily operate the health risk assessment system 100, are stored. The memory device may be, for example, a random access memory (RAM), and other forms of memory used in conjunction with RAM memory, including but not limited to flash memory (FLASH), programmable read only memory (PROM), and electronically erasable programmable read only memory (EEPROM). The health risk assessment computing device 104 that includes a processor-based microcontroller may be integrated with one or more of the environmental sensors 102 and/or one or more of the proximity monitoring assembly 101.

In the embodiment depicted in FIG. 1B, the health risk assessment system 100 includes environmental sensors 102 that are room-level sensors, which are mounted in a room or a closed space and measure environmental data of the area. The proximity monitoring assembly 101 of the health risk assessment system 100 is a proximity wearable device 101 that is wearable on a person, such as being worn with a lanyard or on PPE or being clipped to a belt around the waist of the person. The proximity wearable devices 101 communicate with each other in two-way communication to facilitate proximity detection between any two proximity wearable devices 101. The health risk assessment computing devices 104 of the health risk assessment system 100 include a plurality of user computing devices 110, such as a laptop, a smart phone, or a tablet computer. The user computing devices 110 may function as syncing devices. The proximity wearable device 101 communicates with the syncing device 110. The health risk assessment computing devices 104 further include a server computing device that is cloud-based. The proximity wearable devices 101 are synced to the syncing devices 110, where signals and data detected and/or stored in the proximity wearable devices 101 are sent to the syncing devices 110 and data or commands received by the syncing devices 110 are sent to the proximity wearable devices 101. The syncing devices 110 communicate with the server computing device 108, where data and/or signals stored in the syncing devices are uploaded to the server computing device 108 and the server computing device 108 sends data such as recommendations and alerts and/or commands to the syncing devices 110, in a time interval or in real time. The room-level sensors 102 directly communicate with the server computing device 108 and upload signals and data detected and/or stored in the room-level sensors 102.

In the depicted embodiment, the proximity monitoring assembly 101 includes a proximity sensor 152 (FIG. 1C). The proximity sensor 152 may be a sensor that uses radio waves to detect proximity of objects or a distance between objects. The proximity sensor 152 may be a Bluetooth low energy (BLE) received signal strength indicator (RSSI) that uses RSSI of the BLE radio waves to infer whether people are close together. The proximity sensor 152 acts as a transmitter and a receiver and detects a like device that communicates using the BLE radio waves. The proximity sensor 152 measures proximity to the like device, duration of the interaction, and amount of exposure of external hazards, using the RSSI of the BLE waves and identification of the other device based on information programmed in the BLE radio waves. Alternatively, the proximity sensor may be based on radio frequency identification (RFID), ultra-wideband (UWB), wireless fidelity (WiFi), InfraRed, acoustic signalling, or other methods that enable the proximity sensor to function as described herein.

In the exemplary embodiment, the proximity monitoring assembly 101 further includes an alert device 154 that provides a visual, audio, and/or haptic alert. The alert device 154 may be a visual alert device such as a light-emitting diode (LED) indicator. The alert device 154 may include a haptic motor to produce a haptic alert such as a vibration. The alert device 154 may provide verbal alerts in the form of texts displayed on the alert device 154 or voices played by the alert device 154.

In the exemplary embodiment, the proximity monitoring assembly 101 further includes a power supply 156. The power supply 156 may be a battery that provides electric power to components of the proximity monitoring assembly 101. In some embodiments, the proximity monitoring assembly 101 includes a switch 158 that allows a user to manually turn the power on or off. In some embodiments, the proximity monitoring assembly 101 may further include an accelerometer that measures the acceleration of motion of the wearer or a gyroscope that measures the angular velocity of the wearer. Power of the proximity monitoring assembly 101 may also be turned on/off, or switched to a low power mode or sleep mode, based on analysis of data from the accelerometer/gyroscope to indicate whether the proximity monitoring assembly 101 is in use.

In the exemplary embodiment, the proximity monitoring assembly 101 further includes a proximity monitoring computing device 160. In some embodiments, the proximity monitoring computing device 160 includes a processor-based microcontroller including a processor and a memory device wherein executable instructions, commands, and control algorithms, as well as other data and information needed to satisfactorily operate the proximity monitoring assembly 101, are stored. The memory device may be, for example, a RAM, and other forms of memory used in conjunction with RAM memory, including but not limited to FLASH, PROM, and EEPROM.

The proximity monitoring assembly 101 may communicate with other proximity monitoring assemblies 101 or the health risk assessment computing device 104 through wired or wireless communication. The proximity monitoring assembly 101 may communicate wirelessly through Bluetooth technology using the BLE radio waves emitted by the proximity sensor 152. In other words, the BLE radio waves generated by the proximity sensor 152 are used to detect proximity of the persons, as well as communicating with other devices through the Bluetooth technology. The proximity monitoring assembly 101 may communicate with a mobile device and upload data detected and/or stored in the proximity monitoring computing device 160 to the mobile device. As a result, a person may look up the data on the mobile device. For example, parents may monitor the whereabouts and status of their children by looking up the data uploaded from the proximity monitoring assemblies 101 worn by the children or recommendations provided by the health risk assessment system 100. The functionality and communication of the proximity monitoring assembly 101, however, does not require a use of a mobile device, which is advantageous in environments where a mobile device is not allowed, for example, a hazardous environment.

In some embodiments, the proximity monitoring assemblies 101 do not include the proximity monitoring computing device 160. The proximity sensor 152 directly triggers the alert device 154, and may directly communicate with the health risk assessment computing device 104.

In operation, the environmental sensors 102 measure temperature, humidity, carbon-dioxide levels, and/or other signals and data about the environment. The proximity monitoring assembly 101 measures a distance of the wearer from another wearer of a like proximity monitoring assembly 101. The signals and data stored and detected by the environmental sensors 102 and/or the proximity monitoring assembly 101 are transmitted to the health risk assessment computing devices 104, which determines the health risk posed to the wearer and may transmit a command, recommendation, or alert in response to the detected signals and assessment.

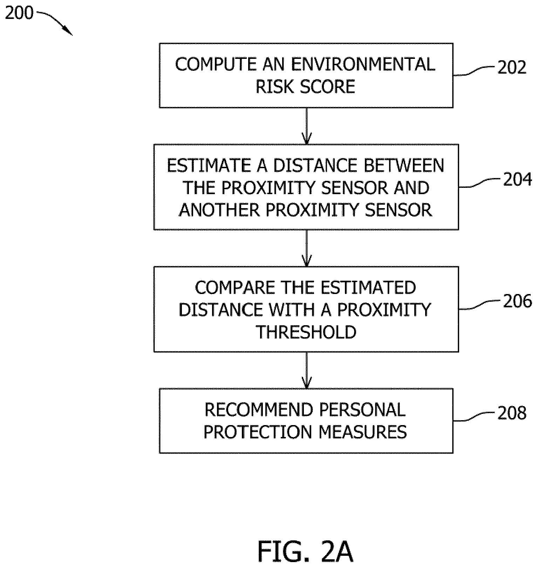

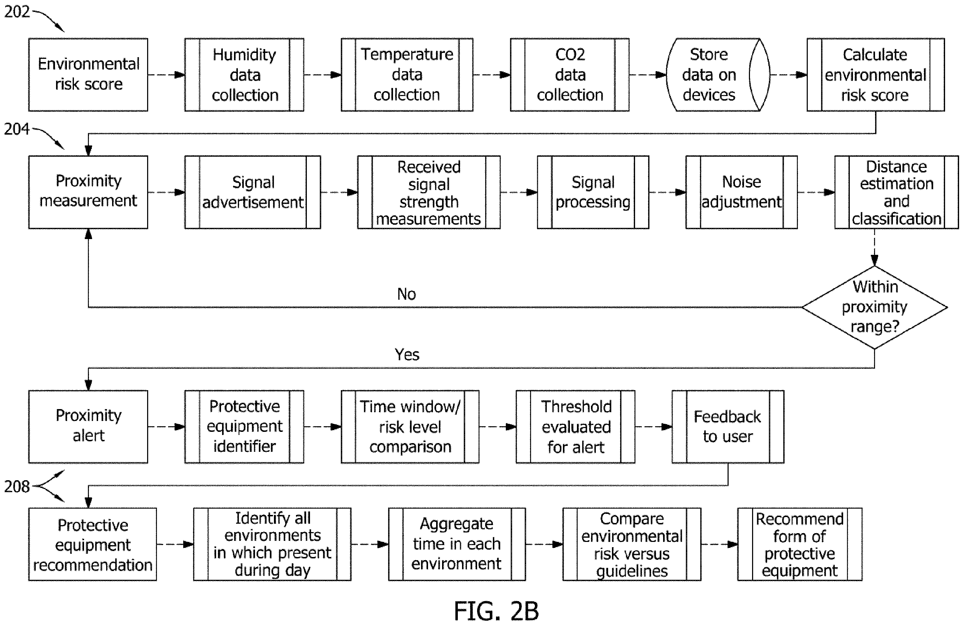

FIGs. 2A and 2B are flow charts of an exemplary method 200 of assessing health risk in an environment. FIG. 2B is a flow chart of an exemplary embodiment of the method 200. The method 200 includes computing 202 an environmental risk score based on environmental data received from at least one environmental sensor. Further, the method 200 includes estimating 204 proximity between one of the at least one proximity sensor and another one of the at least one proximity sensor based on proximity data received from the at least one proximity sensor. The method also includes comparing 206 the estimated distance with a proximity threshold corresponding to the environmental risk score. A proximity threshold as used herein is the threshold of a distance between two individuals, at or farther than which the two individuals should be positioned apart. In some embodiments, the method 200 does not include comparing 206. The method 200 also includes recommending 208 personal protection measures based on the comparison and the environmental risk score. Alternatively, the method 200 includes recommending 208 personal protection measures based on the environmental risk score and the proximity data.

In the depicted embodiment, in computing 202 an environmental risk score, environmental data are collected. Environmental data may include humidity data, temperature data, and/or carbon-dioxide data (FIG. 2B). The level of humidity in the air influences the transmissibility of airborne disease. Virus decay exhibits a U-shaped dependence on relative humidity, where virus decays two to five times faster at 65% relative humidity than at 40% and 100% humidity. On the other hand, in general, at a given temperature, higher levels of humidity result in droplets or aerosols of contagion such as virus being deposited on the ground or surface rapidly, which reduces the chance of the contagion being breathed in from the air. Humidity levels may be detected using a capacitive, resistive, or thermal conductivity sensor. A humidity sensor 114 may be situated on a device that is at a fixed location in a room or area. Alternatively, a humidity sensor 114 is on a wearable device.

Air temperature data may also be collected. Air temperature also influences the transmissibility of airborne disease. In some circumstances, lower temperature may increase the risk of disease transmission. For example, median estimated viral half-life is more than 24 hours at 10 °C (50 °F) with 40% relative humidity, while the half-life is approximately one hour and a half at 27 °C (80 °F) and 65% relative humidity, where virus decays roughly five to ten times faster. A temperature sensor 112 may be situated on a device at a fixed location in a room or area. Alternatively, the temperature sensor 112 may be on a wearable device.

Carbon-dioxide data also may be collected. Carbon-dioxide levels reflect ventilation in an area. The quality of ventilation influences the transmissibility of airborne disease. In general, a higher rate of ventilation tends to reduce the risk of disease transmission. Fresh air that is circulated from outdoors may reduce the transmission risk. The carbon-dioxide sensor 116 may be situated on a device at a fixed location in a room or area, or alternatively, on a wearable device.

The data may be stored on the environmental sensors 102. The data may be transmitted to a server computing device in real time or in a time interval.

Based on the environmental data, an environmental risk score is computed 202. The environmental risk score of an environment is one or more composites of the humidity, temperature, and carbon-dioxide data for the environment, such as a room, a closed space, or an area. The environmental risk score indicates the overall risk level of the environment, i.e., the potential range of concentration of viral particles in the air and the associated risk level.

Besides environmental factors, the risk of an individual of airborne disease also depends on the proximity of people. In the depicted embodiment, proximity is measured and estimated 204 through transmitting and receiving radio frequency signals using a proximity sensor 152 on a proximity monitoring assembly 101 (see FIG. 1C). The proximity monitoring assembly 101 is worn by an individual and monitors the wearer. The wearable proximity monitoring assembly 101 transmits a signal that is detectable by other devices. The signal may be a radio frequency (RF) signal such as BLE radio waves. The RF signal includes an identifier of the transmitting proximity monitoring assembly 101, which allows other devices to identify the transmitting proximity monitoring assembly 101. The proximity monitoring assembly 101 also receives RF signals transmitted from other devices in the locality. The signal strength of the received signals is recorded and stored. The signals may be transmitted to the health risk assessment computing devices in real time or in a time interval. The signals may be processed, such as being filtered with a Kalman filter to improve the signal to noise ratio of the signals or imputing signal values if some packets are not received in the signals. The signals may also be adjusted by RF noise in the environment. For example, feedback from external wireless beacons is used to measure RF noise in the environment. In proximity calculation, the RF noise is removed from the received RF signals. The RF signals are used to estimate a distance of the proximity monitoring assembly 101 from other like devices, where the distance is correlated with the received signal strength index of signals transmitted from other devices.

In some embodiments, a location sensor such as a GPS sensor or a wireless beacon provides location data of an individual. Base on the location data, whether the individual is indoors or outdoors is determined. Being located indoors or outdoors greatly affects transmission risk, where being outdoors tends to reduce the risk, compared to being indoors.

In the exemplary embodiment, recommending 208 personal protection measures includes alerting proximity when the distance between two wearers of the proximity monitoring assemblies is less than a proximity threshold, where a wearer of the proximity assembly is too close to another wearer of the proximity assembly (see FIG. 2B) and should stay farther from another wearer than the proximity threshold. A proximity threshold between wearers of the proximity monitoring assemblies 101 may be adjusted or changed, based on the environmental risk score. For example, if the environmental risk score is higher, for the same risk of airborne disease, the proximity threshold is greater, where individuals should stay farther apart each other. Conversely, if the environmental risk score is lower, for the same risk of airborne disease, the proximity threshold is smaller, where individuals can stay closer together. In some embodiments, because the risk to an individual reduces if the individual is outdoors, for the same environmental risk score, the proximity threshold is greater when the individual is outdoors than when the individual is indoors.

In some embodiments, the PPE configuration of the wearer is determined. The level of protection worn by an individual influences the individual's level of risk. Using near field communication (NFC), identification tags such as RFID may be used to identify the type of PPE that is worn on the individual. In one example, the manufacture date or expiration date of the PPE may be captured and sent to the health risk assessment computing device 104 in its risk analysis. The health risk assessment computing device 104 may determine whether the PPE is effective or expired based on the manufacture data or the expiration date. The wearer may enter the PPE configuration, such as the type of the PPE and the manufacture/expiration date, if the PPE lacks an identifier that allows automatic detection of the PPE configuration. The PPE configuration may be defaulted to no PPE being worn as the baseline for the PPE configuration.

In some embodiments, recommending 208 personal protection measures includes generating an alert when the risk is above a risk threshold. Because PPE reduces the risk of airborne disease and PPE configuration affects the degree of reduction in the risk, a risk threshold depends on the PPE configuration of the wearer of the proximity monitoring assembly 101, as well as on the environmental data and proximity data. The risk threshold may be determined based on the PPE configuration, the environmental data, and the proximity data. In one example, the risk of airborne disease is estimated as an accumulated risk score. To compute an accumulated risk score, the risk score of the wearer is accumulated across a predefined time window or period of time to derive the accumulated risk score. The risk score at a given time point depends on and may be computed as a function of the environmental data, proximity data, location data, or a combination thereof. For example, the risk score may be calculated as being proportional to the environmental risk score and inversely proportional to the proximity data, and may be reduced by a factor when the individual is outdoors. In another example, the risk score may be calculated using a Wells-Riley model, where the risk score is inversely related to the ventilation rate, measured in meters cubed per second. A risk model that defines how a risk score accumulates in time is used to estimate the accumulated risk score. In close proximity situations, the accumulated risk score increases over time. In situations where the wearers are not in proximity with each other, the accumulated risk score may stay flat or decrease over time because the virus decays.

The following is an example of estimating an environmental risk score and accumulated risk score based on the environmental risk score/environmental data and proximity data. An environmental risk score may be estimated based on estimated airborne viral load. A viral load is the concentration of viral particles in the air, and is proportional to the probability of airborne transmission of the virus. The higher viral load, the probability is higher. Viral load may be estimated based on environmental data such as humidity, temperature, carbon dioxide levels. Viral half-life or half-life of the virus indicates infectivity of the virus, and is a determinant of viral load. Viral half-life is a measurement of viral decay rate $k$ as the time taken for virus infectivity to reduce by half. In an exemplary model, the higher the viral half-life, the higher the anticipated viral load.

In the exemplary embodiment, the baseline risk of viral transmission in an indoor environment may be established from medical guidelines. For example, individuals are commonly advised to avoid spending 15 minutes within 1.8 meters (6 feet) of another person, as this is perceived as a significant threshold for COVID-19 transmission. This default setting of 15 minutes at 1.8 meters (6 feet) may be adjusted based on the environmental risk, calculated with data from environmental sensors. In other words, the risk or risk score in the setting of 15 minutes indoors at 1.8 meters (6 feet) is the baseline and may be assigned a baseline value or a default value, and environmental risk scores and risk scores of an individual at various environments are estimated based on the relationships and effects of environmental data and proximity data on viral load and/or risk of airborne transmission. For example, the relationship between viral decay and temperature may be characterized by the Arrhenius equation as shown below:

$$k = A.\exp\left(-Ea/RT\right), \qquad\qquad (1)$$

which describes a viral decay rate k dependent on activation energy (Ea), the universal gas constant (R), an asymptotic high-temperature reaction rate (A), and the absolute temperature (T).

For humidity, an empirically observed U-shaped relationship with viral half-life may be used to estimate the airborne viral load. In some embodiments, the sensor readings are translated into the viral half-life via a plot resembling a parabolic curve. Equation (2) below may be used to approximate the half-life of the virus $T_{1/2}$ in an environment having a relative humidity (RH) ranging from 50% to 90% at a temperature of 10 °C:

$$T_{1/2} = 0.00672x^2 - 0.767x + 22.656\,, \qquad\qquad (2)$$

where x is the RH. As such, at RH of 90% the expected viral half-life at the temperature of 10 °C is 8 hours. At higher temperatures the curve may be transposed to lower half-life values by adjusting the constant values in the above Eqn. (2).

The environmental risk score depends on the expected viral half-life. The expected viral half-life may influence the environmental risk score via fixed thresholds, a linear relationship, or using a statistical distribution such as a sigmoid statistical relationship between half-life and an environmental risk score. A sigmoid distribution imposes a stronger relationship between estimated viral half-life and an environmental risk score at moderate viral half-life levels, while the relationship is weaker at low and high viral half-life levels respectively.

In an indoor setting, when the environmental sensors indicate an environment of 22 °C and 40% RH, the median half-life may be around 6.4 hours. In an environment with a room temperature of 27 °C and a RH of 40%, the median half-life may drop to around 3.4 hours. By applying a linear relationship, or assuming a linear portion of the statistical distribution between the environmental risk score and the viral half-life at this range of RH, changing from an environment of 27 °C and 40% RH to an environment of 22 °C and 40% RH results in an 88% increase in the environmental risk score. For example, defining an environmental risk score that may range from 0 to 1.0, and a default of 0.5, this may increase the environmental risk score to 0.94. As the viral half-life increases beyond the linear portion of the sigmoid distribution, the rate of increase of environmental risk score would be reduced, i.e. the curve is asymptotic to 1.0.

In the exemplary embodiment, for the proximity risk, proximity of 1.8 meters (6 feet) may be assigned a risk of 0.5, and the proximity risk reaches 1.0 when the proximity is less than or equal to 0.3 meter (1 foot). In this example, the proximity risk score increases linearly between 1.8 meters (6 feet) and 0.3 meter (1 foot). Any distance outside of 3.7 meters (12 feet) may be considered a risk of zero, and the proximity risk score falls linearly between 1.8 meters (6 feet) and 3.7 meters (12 feet).

In the exemplary embodiment, multiplying the environmental risk score by the proximity risk gives the risk score of an individual for this time point (one minute duration in this example), i.e. (0.94 * 0.5) = 0.47 risk score. At subsequent time points, risk scores are summed to give an aggregate risk score. An aggregate risk score of 3.75 corresponds to 1.8 meters (6 feet) distance for 15 minutes. Therefore, when aggregate risk reaches 3.75 an alert is triggered, where the individual has spent 15 minutes at proximity of 1.8 meters (6 feet). In the above example, the risk score of the individual increases from the default value of (0.5*0.5) = 0.25 risk score to 0.47 risk score for each minute, and the aggregate risk score reaches the threshold of 3.75 in 7.98 minutes, i.e. an alert will be generated on the eighth data point (of one minute duration each). The risk score for each time point will reflect changes in both the proximity risk and the environmental risk. If the sampling rate is greater than one per minute, the magnitude of each risk score drops commensurate to the change in time interval.

As another example, in an outdoors setting with a temperature of 10 °C and RH of 65%, the median half-life of the virus may be 14.2 hours. When the sensor indicates that the RH has increased to 85%, the median half-life may drop to 13.8 hours. The viral half-life may be mapped onto the environment risk score, which is then multiplied by the proximity risk at each time point, to calculate the aggregate risk score. Furthermore, the estimated risk of viral transmission indoors may be adjusted based on carbon dioxide sensor readings. Carbon dioxide levels of 600-800 parts per million (PPM) are typical in well-ventilated office environments, while outdoor levels are typically lower at approximately 300-350 PPM, although there is variation. A level above 750 PPM may be considered to elevate the risk of transmission of disease, although levels above 2,000 have been noted in some disease outbreaks. Indoor $CO_2$ levels of 600 PPM or less tend to reflect high quality ventilation. The reading of the $CO_2$ sensor, relative to the volume mixing ratio of the excess $CO_2$ that an uninfected person inhales for one hour in the environment, multiplied by 0.01%, gives the probability of infection over one hour.

If the accumulated risk score is above the risk threshold, an alert is triggered. The alert may include a notification that PPE should be worn or the PPE is inadequate to protect against the risk of airborne disease, such as whether the type of the PPE is adequate to protect the wearer of the PPE from the risk or whether the PPE is effective or expired. The alert may be sent to a user, such as the wearer of the proximity monitoring assembly 101, an administrator, a supervisor, or management. The alert may be sent to the proximity monitoring assembly 101 or sent to a computing device such as a mobile device, a laptop computer, or a desktop computer.

In the depicted embodiment, recommending 208 personal protection measures may further include providing recommendation on PPE. For example, all environments in which an individual is present throughout a period of time, such as a day, are identified. A record of the environments may be stored in a database. Aggregate time the individual spent in each environment is determined for the period of time. An aggregate environmental risk score is computed as an aggregation of the environmental risk scores in all environments for the period of time. The level of aggregate environmental risk score is compared against clinical guidelines or organization deadlines, to determine an appropriate form of PPE. For example, if the ventilation level of a building, a room or an area is optimal, the recommendation may be a face shield or a face mask. If the ventilation level is relatively poor, the recommendation may be a face mask. The recommendation may be provided to a user. The user may be the wearer of the proximity monitoring assembly 101, an administrator, a supervisor, or management. The recommendation may be provided via an application on a computing device, such as a mobile phone, laptop, desktop, or tablet.

The health risk assessment system 100 may be used in a harsh and/or hazardous environment. In addition to assessing health risk associated with airborne infection of contagions, other health risks associated with a harsh and/or hazardous environment is assessed.

For example, a risk of heat stress is assessed in high temperature areas such as areas in factories that are near furnaces. The environmental sensors may characterize the risk of heat stress and alert staff members of hazardous working conditions. In assessing the heat stress, the health risk assessment computing device 104 considers the effects of wearing of PPE and types of PPE on heat stress.

In another example, factories are required to provide adequate ventilation to protect workers from dangerous emissions such as fumes from certain industrial processes or engine. Alerting hazardous environment is particularly important in harsh and hazardous environments such as mines. The monitoring of carbon-dioxide level may be used to alert staff members of sub-optimal ventilation with the potential presence of dangerous emissions. A threshold for the optimal ventilation may be higher for a harsh and hazardous environment than a typical environment.

In yet another example, besides influencing the viral load, humidity levels also affect the remaining life of machinery in industrial settings. Steam and humidity are byproducts of many industrial processes, which lead to buildup of condensation or even mold that may harm the machinery or even the building. An alert may be triggered if the humidity level exceeds a threshold.

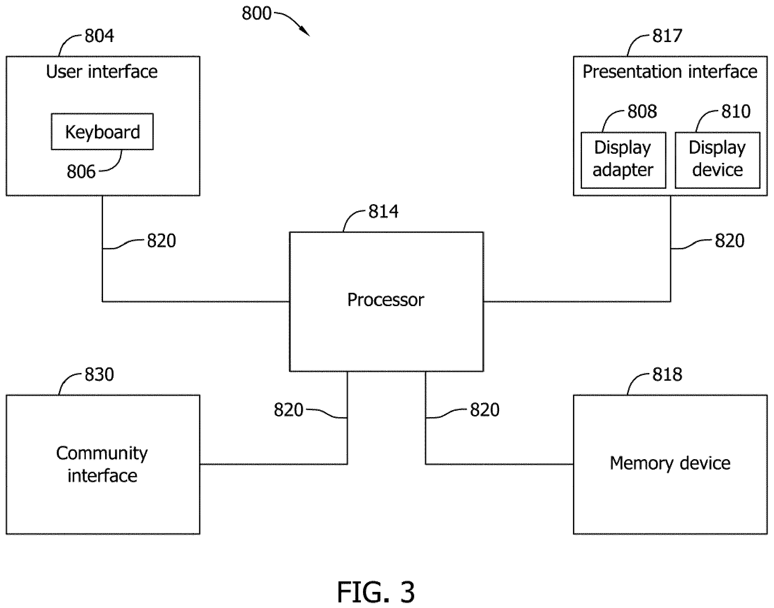

The health risk assessment computing device 104 described herein may be any suitable user computing device 800 and software implemented therein. FIG. 3 is a block diagram of an exemplary computing device 800. In the exemplary embodiment, the computing device 800 includes a user interface 804 that receives at least one input from a user. The user interface 804 may include a keyboard 806 that enables the user to input pertinent information. The user interface 804 may also include, for example, a pointing device, a mouse, a stylus, a touch sensitive panel (e.g., a touch pad and a touch screen), a gyroscope, an accelerometer, a position detector, and/or an audio input interface (e.g., including a microphone).

Moreover, in the exemplary embodiment, computing device 800 includes a display interface 817 that presents information, such as input events and/or validation results, to the user. The display interface 817 may also include a display adapter 808 that is coupled to at least one display device 810. More specifically, in the exemplary embodiment, the display device 810 may be a visual display device, such as a cathode ray tube (CRT), a liquid crystal display (LCD), a light-emitting diode (LED) display, and/or an "electronic ink" display. Alternatively, the display interface 817 may include an audio output device (e.g., an audio adapter and/or a speaker) and/or a printer.

The computing device 800 also includes a processor 814 and a memory device 818. The processor 814 is coupled to the user interface 804, the display interface 817, and the memory device 818 via a system bus 820. In the exemplary embodiment, the processor 814 communicates with the user, such as by prompting the user via the display interface 817 and/or by receiving user inputs via the user interface 804. The term "processor" refers generally to any programmable system including systems and microcontrollers, reduced instruction set computers (RISC), complex instruction set computers (CISC), application specific integrated circuits (ASIC), programmable logic circuits (PLC), and any other circuit or processor capable of executing the functions described herein. The above examples are exemplary only, and thus are not intended to limit in any way the definition and/or meaning of the term "processor."

In the exemplary embodiment, the memory device 818 includes one or more devices that enable information, such as executable instructions and/or other data, to be stored and retrieved. Moreover, the memory device 818 includes one or more computer readable media, such as, without limitation, dynamic random access memory (DRAM), static random access memory (SRAM), a solid state disk, and/or a hard disk. In the exemplary embodiment, the memory device 818 stores, without limitation, application source code, application object code, configuration data, additional input events, application states, assertion statements, validation results, and/or any other type of data. The computing device 800, in the exemplary embodiment, may also include a communication interface 830 that is coupled to the processor 814 via the system bus 820. Moreover, the communication interface 830 is communicatively coupled to data acquisition devices.

In the exemplary embodiment, the processor 814 may be programmed by encoding an operation using one or more executable instructions and providing the executable instructions in the memory device 818. In the exemplary embodiment, the processor 814 is programmed to select a plurality of measurements that are received from data acquisition devices.

In operation, a computer executes computer-executable instructions embodied in one or more computer-executable components stored on one or more computer-readable media to implement aspects of the disclosure described and/or illustrated herein. The order of execution or performance of the operations in embodiments of the disclosure illustrated and described herein is not essential, unless otherwise specified. That is, the operations may be performed in any order, unless otherwise specified, and embodiments of the disclosure may include additional or fewer operations than those disclosed herein. For example, it is contemplated that executing or performing a particular operation before, contemporaneously with, or after another operation is within the scope of aspects of the disclosure.

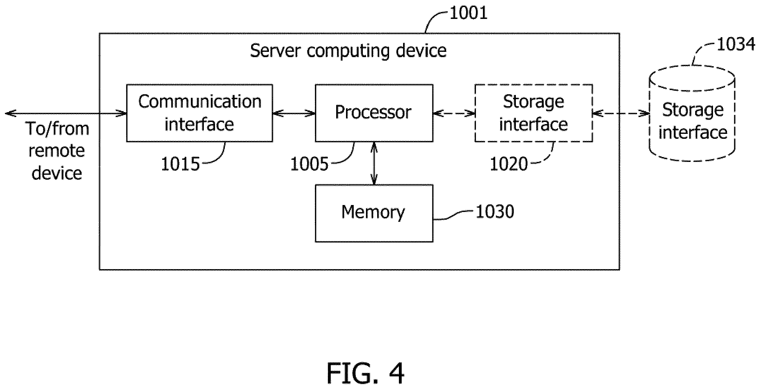

FIG. 4 illustrates an exemplary configuration of a server computing device 1001 such as the cloud-based server computing device 108. The server computing device 1001 also includes a processor 1005 for executing instructions. Instructions may be stored in a memory area 1030, for example. The processor 1005 may include one or more processing units (e.g., in a multi-core configuration).

The processor 1005 is operatively coupled to a communication interface 1015 such that the server computing device 1001 is capable of communicating with a remote device such as the proximity monitoring computing device 160, environmental sensors 102, or another server computing device 1001. For example, the communication interface 1015 may receive data from the proximity monitoring computing device 160 and the environmental sensors 102, via the Internet.

The processor 1005 may also be operatively coupled to a storage device 1034. The storage device 1034 is any computer-operated hardware suitable for storing and/or retrieving data, such as, but not limited to, wavelength changes, temperatures, humidity, carbon dioxide levels, and/or proximity. In some embodiments, the storage device 1034 is integrated in the server computing device 1001. For example, the server computing device 1001 may include one or more hard disk drives as the storage device 1034. In other embodiments, the storage device 1034 is external to the server computing device 1001 and may be accessed by a plurality of server computing devices 1001. For example, the storage device 1034 may include multiple storage units such as hard disks and/or solid state disks in a redundant array of inexpensive disks (RAID) configuration. The storage device 1034 may include a storage area network (SAN) and/or a network attached storage (NAS) system.

In some embodiments, the processor 1005 is operatively coupled to the storage device 1034 via a storage interface 1020. The storage interface 1020 is any component capable of providing the processor 1005 with access to the storage device 1034. The storage interface 1020 may include, for example, an Advanced Technology Attachment (ATA) adapter, a Serial ATA (SATA) adapter, a Small Computer System Interface (SCSI) adapter, a RAID controller, a SAN adapter, a network adapter, and/or any component providing the processor 1005 with access to the storage device 1034.

At least one technical effect of the systems and methods described herein includes (a) calculation and adjustment of a proximity threshold based on environmental data, proximity data, and/or location data; (b) assessment of health risk in an environment based on environmental data, proximity data, and/or location data; (c) calculating a risk threshold based on environmental risk data, proximity data, and the PPE configuration; (d) provision of recommendation on PPE based on an aggregate environmental score in all environments that an individual is in during a period of time; and (e) assessment of a health risk to an individual in a harsh and hazardous environment based on environmental data and/or proximity data.

The benefits and advantages of the inventive concepts are now believed to have been amply illustrated in relation to the exemplary embodiments disclosed.

An embodiment of a method of assessing health risk in an environment is disclosed. The method is implemented with at least one health risk assessment computing device in communication with at least one environmental sensor and at least one proximity monitoring assembly each including a proximity sensor. The method includes computing an environmental risk score based on environmental data received from the at least one environmental sensor, and estimating a distance between the at least one proximity monitoring assembly and another proximity monitoring assembly based on proximity data received from the at least one proximity monitoring assembly. The method also includes comparing the estimated distance with a proximity threshold corresponding to the environmental risk score, and recommending personal protection measures based on the comparison and the environmental risk score.

Optionally, computing an environmental risk score further includes estimating a viral load based on the environmental data; and computing the environmental risk score based on the viral load. Recommending personal protection measures includes generating an alert that a wearer of one of the at least one proximity monitoring assembly is too close to a wearer of another one of the at least one proximity monitoring assembly when the estimated distance is smaller than the proximity threshold. Estimating a distance further includes receiving location data of a wearer of one of the at least one proximity monitoring assembly, the method further including determining the wearer is indoors or outdoors based on the location data and adjusting the proximity threshold based on the determination. The method further includes estimating an accumulated risk score of a wearer of one of the at least one proximity monitoring assembly during a period of time, based on computed environmental risk scores and estimated distance over the period of time. The method further includes computing a risk threshold based on the accumulated risk score, the estimated distance, and a configuration of personal protection equipment (PPE) worn by the wearer, and recommending personal protection measures further including alerting the wearer a health risk if the accumulated risk score is higher than the computed risk threshold. Estimating a distance further includes receiving information on PPE worn by a wearer of one of the at least one proximity monitoring assembly, and recommending personal protection measures further including providing a notification whether the PPE is adequate to protect against a risk of airborne disease based on the accumulated risk score. Receiving information further includes determining a type of the PPE, and recommending personal protection measures further includes recommending whether the type of the PPE is adequate to protect against the risk of airborne disease based on the accumulated risk score. Alternatively, receiving information further includes determining an expiration date of the PPE, and recommending personal protection measures further includes recommending whether the PPE is effective or expired in protecting against the risk of airborne disease based on the accumulated risk score. The method further includes estimating an aggregate environmental risk score of an individual by computing an aggregation of environmental risk scores in all environments that the individual is in during a period of time, and recommending personal protection measures further including recommending PPE for the individual based on the aggregate environmental risk score. The at least one health risk assessment computing device further includes a server computing device and at least one syncing device, and estimating a distance further including syncing each of the at least one proximity monitoring assembly to one of the at least one syncing device, and communicating the server computing device with the at least one syncing device. Computing an environmental risk score further includes receiving at least one of i) humidity data, ii) temperature data, and iii) carbon-dioxide data. Alternatively, computing an environmental risk score further includes receiving the humidity data, the temperature data, and the carbon-dioxide data. Wearers of the at least one proximity monitoring assembly are in a harsh and hazardous environment. The method further includes estimating a risk of heat stress in the harsh and hazardous environment based on the temperature data. The method further includes estimating a carbon-dioxide level based on the carbon-dioxide data, and determining whether ventilation at the harsh and hazardous environment is sufficient to protect workers against emissions in the harsh and hazardous environment. The method further includes estimating a humidity level based on the humidity data, and providing an alert when the humidity level exceeds a threshold.

An embodiment of a health risk assessment system of assessing health risk in an environment is provided. The system includes at least one environmental sensor, at least one proximity monitoring assembly each including a proximity sensor, and at least one health risk assessment computing device in communication with the at least one environment sensor and the at least one proximity monitoring assembly. The at least one health risk assessment computing device is programmed to compute an environmental risk score based on environmental data received from the at least one environmental sensor, and estimate a distance between one of the at least one proximity monitoring assembly and another one of the at least one proximity monitoring assembly based on proximity data received from the at least one proximity monitoring assembly. The at least one health risk assessment computing device is also programmed to recommend personal protection measures based on the environmental risk score and the proximity data.

Optionally, the at least one health risk assessment computing device is further programmed to estimate an accumulated risk score of a wearer of one of the at least one proximity monitoring assembly during a period of time based on computed environmental risk scores and estimated distance over the period of time, compute a risk threshold based on the accumulated risk score, the estimated distance, and a configuration of personal protection equipment (PPE) worn by the wearer, and recommend personal protection measures based on the estimated accumulated risk score and the computed risk threshold. The health risk assessment computing device is further programmed to estimate an aggregate environmental risk score of an individual by computing an aggregation of environmental risk scores in all environments that the individual is in during a period of time, and recommend PPE for the individual based on the aggregate environmental risk score.

While exemplary embodiments of components, assemblies and systems are described, variations of the components, assemblies and systems are possible to achieve similar advantages and effects. Specifically, the shape and the geometry of the components and assemblies, and the relative locations of the components in the assembly, may be varied from that described and depicted without departing from inventive concepts described. Also, in certain embodiments certain components in the assemblies described may be omitted to accommodate particular types of connectors and supports, or the needs of particular installations, while still providing cost effective connector assemblies for electrical wiring or cabling.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

**Claims**

1. A method of assessing personal health risk in an environment, implemented with at least one health risk assessment computing device in communication with at least one environmental sensor and at least one wearable proximity monitoring assembly each including a proximity sensor, the method comprising:

   computing an environmental risk score based on environmental data received from the at least one environmental sensor;
   estimating a distance between the at least one proximity monitoring assembly and another proximity monitoring assembly based on proximity data received from the at least one proximity monitoring assembly; and
   comparing the estimated distance with a proximity threshold corresponding to the environmental risk score; and recommending personal protection measures based on the comparison and the environmental risk score.

2. The method of claim 1,
   wherein computing an environmental risk score further comprises:

   estimating a viral load based on the environmental data; and
   computing the environmental risk score based on the viral load; and/or
   wherein recommending personal protection measures comprises generating an alert that a wearer of one of the at least one proximity monitoring assembly is too close to a wearer of another one of the at least one proximity monitoring assembly when the estimated distance is smaller than the proximity threshold.

3. The method of claim 2, wherein estimating a distance further comprises receiving location data of a wearer of one of the at least one proximity monitoring assembly, the method further comprising:

   determining the wearer is indoors or outdoors based on the location data; and
   adjusting the proximity threshold based on the determination.

4. The method of claim 1, further comprising:
   estimating an accumulated risk score of a wearer of one of the at least one proximity monitoring assembly during a period of time, based on computed environmental risk scores and estimated distance over the period of time.

5. The method of claim 4, further comprising:

   computing a risk threshold based on the accumulated risk score, the estimated distance, and a configuration of personal protection equipment (PPE) worn by the wearer; and
   recommending personal protection measures further comprising alerting the wearer a health risk if the accumulated risk score is higher than the computed risk threshold

6. The method of claim 4, wherein estimating a distance further comprises:

   receiving information on PPE worn by a wearer of one of the at least one proximity monitoring assembly; and
   recommending personal protection measures further comprising providing a notification whether the PPE is adequate to protect against a risk of airborne disease based on the accumulated risk score.

7. The method of claim 6, wherein receiving information further comprises determining a type of the PPE, and recommending personal protection measures further comprises recommending whether the type of the PPE is adequate to protect against the risk of airborne disease based on the accumulated risk score; and/or
   wherein receiving information further comprises determining an expiration date of the PPE, and recommending personal protection measures further comprises recommending whether the PPE is effective or expired in protecting against the risk of airborne disease based on the accumulated risk score.

8. The method of claim 1, further comprising:

   estimating an aggregate environmental risk score of an individual by computing an aggregation of environmental risk scores in all environments that the individual is in during a period of time; and
   recommending personal protection measures further comprising recommending PPE for the individual based on the aggregate environmental risk score.

9. The method of claim 1, wherein the at least one health risk assessment computing device further comprises a server computing device and at least one syncing device, and estimating a distance further comprising:

   syncing each of the at least one proximity monitoring assembly to one of the at least one syncing device; and
   communicating the server computing device with the at least one syncing device.

10. The method of claim 1, wherein computing an environmental risk score further comprises receiving at least one of i) humidity data, ii) temperature data, and iii) carbon-dioxide data.

11. The method of claim 10, wherein computing an environmental risk score further comprises receiving the humidity data, the temperature data, and the carbon-dioxide data.

12. The method of claim 10, wherein wearers of the at least one proximity monitoring assembly are in a harsh and hazardous environment.

13. The method of claim 12,

    wherein the method further comprises:

    estimating a risk of heat stress in the harsh and hazardous environment based on the temperature data; and/or
    wherein the method further comprises:
    estimating a carbon-dioxide level based on the carbon-dioxide data; and
    determining whether ventilation at the harsh and hazardous environment is sufficient to protect workers against emissions in the harsh and hazardous environment; and/or

    wherein the method further comprises:

    estimating a humidity level based on the humidity data; and
    providing an alert when the humidity level exceeds a threshold.

14. A health risk assessment system of assessing personal health risk in an environment, comprising:

    at least one environmental sensor;
    at least one wearable proximity monitoring assembly each including a proximity sensor; and
    at least one health risk assessment computing device in communication with the at least one environment sensor and the at least one proximity monitoring assembly, the at least one health risk assessment computing device programmed to:

    compute an environmental risk score based on environmental data received from the at least one environmental sensor;
    estimate a distance between one of the at least one proximity monitoring assembly and another one of the at least one proximity monitoring assembly based on proximity data received from the at least one proximity monitoring assembly; and

recommend personal protection measures based on the environmental risk score and the proximity data.

**15.** The health risk assessment system of claim 14,

wherein the at least one health risk assessment computing device is further programmed to:

estimate an accumulated risk score of a wearer of one of the at least one proximity monitoring assembly during a period of time based on computed environmental risk scores and estimated distance over the period of time;
compute a risk threshold based on the accumulated risk score, the estimated distance, and a configuration of personal protection equipment (PPE) worn by the wearer; and
recommend personal protection measures based on the estimated accumulated risk score and the computed risk threshold; and/or

wherein the health risk assessment computing device is further programmed to:

estimate an aggregate environmental risk score of an individual by computing an aggregation of environmental risk scores in all environments that the individual is in during a period of time; and
recommend PPE for the individual based on the aggregate environmental risk score.

EP 4 036 929 A1

FIG. 1A

FIG. 1B

EP 4 036 929 A1

FIG. 1C

EP 4 036 929 A1

200

COMPUTE AN ENVIRONMENTAL
RISK SCORE ⎯202

ESTIMATE A DISTANCE BETWEEN
THE PROXIMITY SENSOR AND
ANOTHER PROXIMITY SENSOR ⎯204

COMPARE THE ESTIMATED
DISTANCE WITH A PROXIMITY
THRESHOLD ⎯206

RECOMMEND PERSONAL
PROTECTION MEASURES ⎯208

FIG. 2A

EP 4 036 929 A1

202

| Environmental risk score | ⟶ | Humidity data collection | ⟶ | Temperature data collection | ⟶ | CO2 data collection | ⟶ | Store data on devices | ⟶ | Calculate environmental risk score |

204

| Proximity measurement | ⟶ | Signal advertisement | ⟶ | Received signal strength measurements | ⟶ | Signal processing | ⟶ | Noise adjustment | ⟶ | Distance estimation and classification |

No

Within proximity range?

Yes

| Proximity alert | ⟶ | Protective equipment identifier | ⟶ | Time window/ risk level comparison | ⟶ | Threshold evaluated for alert | ⟶ | Feedback to user |

208

| Protective equipment recommendation | ⟶ | Identify all environments in which present during day | ⟶ | Aggregate time in each environment | ⟶ | Compare environmental risk versus guidelines | ⟶ | Recommend form of protective equipment |

FIG. 2B

FIG. 3

FIG. 4

EP 4 036 929 A1

Europäisches
Patentamt

European
Patent Office

Office européen
des brevets

# EUROPEAN SEARCH REPORT

**Application Number**

EP 22 15 4070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/012448 A1 (BARAK DROR [IL] ET AL) 14 January 2021 (2021-01-14) * abstract * * paragraph [0020] – paragraph [0032] * * paragraph [0055] – paragraph [0064] * ----- | 1-15 | INV. G16H40/20 G16H50/30 G16H50/80 |
| X | WO 2019/234569 A1 (3M INNOVATIVE PROPERTIES CO [US]) 12 December 2019 (2019-12-12) * abstract * * paragraph [0023] – paragraph [0045] * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 17 June 2022 | Menschner, Philipp |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 15 4070

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2022

| Patent document cited in search report | | Publication date | Patent family member(s) | | Publication date |
|---|---|---|---|---|---|
| US 2021012448 | A1 | 14-01-2021 | US | 2020193341 A1 | 18-06-2020 |
| | | | US | 2021012448 A1 | 14-01-2021 |
| | | | US | 2022012655 A1 | 13-01-2022 |
| | | | WO | 2019058379 A1 | 28-03-2019 |
| WO 2019234569 | A1 | 12-12-2019 | BR | 112020024771 A2 | 23-03-2021 |
| | | | CN | 112236788 A | 15-01-2021 |
| | | | EP | 3803733 A1 | 14-04-2021 |
| | | | KR | 20210015942 A | 10-02-2021 |
| | | | US | 2021233654 A1 | 29-07-2021 |
| | | | WO | 2019234569 A1 | 12-12-2019 |

EPO FORM P0459

For more details about this annex : see Official Journal of the European Patent Office, No. 12/82